(19) Europäisches Patentamt
European Patent Office
Office européen des brevets

(11) Veröffentlichungsnummer: **0 162 384**
**B1**

# (12) EUROPÄISCHE PATENTSCHRIFT

(45) Veröffentlichungstag der Patentschrift: **07.01.87**

(51) Int. Cl.⁴: **C 07 C 69/16**, C 07 C 67/28

(21) Anmeldenummer: **85105764.6**

(22) Anmeldetag: **10.05.85**

## (54) Verfahren zur Herstellung von 2-Alkyl-4,4-diacyloxy-2-butenalen.

(30) Priorität: **19.05.84 DE 3418747**

(43) Veröffentlichungstag der Anmeldung:
**27.11.85 Patentblatt 85/48**

(45) Bekanntmachung des Hinweises auf die Patenterteilung:
**07.01.87 Patentblatt 87/2**

(84) Benannte Vertragsstaaten:
**BE CH DE FR GB IT LI NL**

(56) Entgegenhaltungen:
**EP - A - 0 068 372**
**EP - A - 0 089 585**
**EP - A - 0 089 586**

(73) Patentinhaber: **BASF Aktiengesellschaft, Carl-Bosch-Strasse 38, D-6700 Ludwigshafen (DE)**

(72) Erfinder: **Fischer, Rolf, Dr., Bergstrasse 98, D-6900 Heidelberg (DE)**
Erfinder: **Paust, Joachim, Dr., Ringstrasse 3, D-6708 Neuhofen (DE)**

EP 0 162 384 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 2-Alkyl-4,4-diacyloxy-2-butenalen (2-Alkyl-fumardialdehyd-4-monoacylalen) durch Umsetzung von 2-Alkyl-1,4-diacyloxy-1,3-butadienen mit Sauerstoff oder Sauerstoff übertragenden Verbindungen in Gegenwart von sauren Ionenaustauschern.

2-Alkyl-4,4-diacyloxy-2-butenale lassen sich z.B. nach den Angaben der EP-PS 68 372 durch Umsetzung von 2-Alkyl-1,4-diacyloxy-1,3-butadienen mit Sauerstoff oder Sauerstoff übertragenden Verbindungen in Gegenwart von Carbonsäuren oder nach dem in der EP-PS 89 585 beschriebenen Verfahren durch Erhitzen von 2-Alkyl-2,4-diacyloxy-3-butenalen in Carbonsäuren herstellen. Es ist auch bekannt, dass man 2-Alkyl-2,4-diacyloxy-3-butenale durch Umsetzung von 2-Alkyl-1,4-diacyloxy-1,3-butadienen mit Percarbonsäuren in inerten Lösungsmitteln herstellen kann (EP-PS 89 586). Ferner ist auch bekannt, dass 2-Alkyl-4,4-diacyloxy-2-butenale bei der Behandlung mit sauer wirkenden Verbindungen, wie Mineralsäuren, sauren Ionenaustauschern oder Wasser enthaltenden aliphatischen Carbonsäuren in 3-Alkyl-2,5-dihydrofuran-2-one übergehen (DE-OS 32 10 705).

Nach den genannten Verfahren zur Herstellung von 2-Alkyl-4,4-diacyloxy-2-butenalen erhält man zwar Reaktionsgemische, die als Hauptprodukt das gewünschte trans-2-Alkyl-4,4-diacyloxy-2-butenal enthalten. Von Nachteil ist jedoch, dass sie zusätzlich nennenswerte Mengen an 2-Alkyl-2,4-diacyloxy-3-butenalen und cis-2-Alkyl-4,4-diacyloxy-2-butenalen enthalten können, so dass eine aufwendige und verlustreiche Trennung des Reaktionsgemisches, z.B. durch fraktionierende Destillation nötig werden kann. Wie die Beispiele 1 und 2 der EP-PS 89 585 zeigen, führt auch die nachträgliche Isomerisierung von 2-Alkyl-2,4-diacyloxy-3-butenalen zu 2-Alkyl-4,4-diacyloxy-2-butenalen, bedingt durch die Bildung von 3-Alkyl-2,5-dihydrofuran-2-onen, zu Verlusten an Wertprodukt.

Es bestand daher die Aufgabe, ein Verfahren zur Herstellung von 2-Alkyl-4,4-diacyloxy-2-butenalen zu finden, das die genannten Nachteile nicht besitzt und bei dem möglichst hohe Gehalte an trans-Isomeren erhalten werden. Nur trans-2-Alkyl-4,4-diacyloxy-2-butenale sind nämlich als Ausgangsstoffe für Terpensynthesen geeignet.

Es wurde nun gefunden, dass man 2-Alkyl-4,4-diacyloxy-2-butenale der Formel

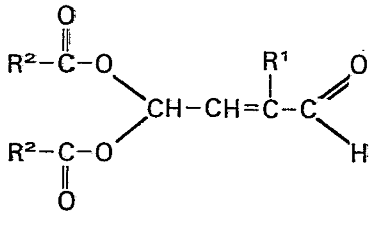

I,

in der $R^1$ einen Alkylrest mit 1 bis 5 Kohlenstoffatomen und $R^2$ ein Wasserstoffatom, einen aliphatischen Rest mit 1 bis 15 Kohlenstoffatomen, einen cycloaliphatischen Rest mit 5 bis 7 Kohlenstoffatomen oder einen aromatischen Rest bedeuten, durch Umsetzung von 2-Alkyl-1,4-diacyloxy-1,3-butadienen der Formel

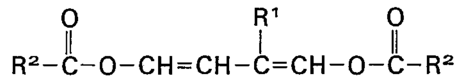

II,

in der $R^1$ und $R^2$ die obengenannte Bedeutung besitzen, in einem Lösungsmittel mit Sauerstoff oder Sauerstoff übertragenden Verbindungen, dadurch besonders vorteilhaft herstellen kann, dass man die Umsetzung in Gegenwart von sauren Ionenaustauschern durchführt.

Nach dem neuen Verfahren werden die 2-Alkyl-4,4-diacyloxy-2-butenale aus den 2-Alkyl-1,4-diacyloxy-1,3-butadienen mit hoher Selektivität und überwiegend als trans-Isomere erhalten.

Die Reaktion lässt sich für den Fall der Herstellung von trans-2-Methyl-4,4-diacetoxy-2-butenal aus 2-Methyl-1,4-diacetoxy-1,3-butadien in Eisessig als Lösungsmittel formal durch die folgenden Formeln anschaulich machen:

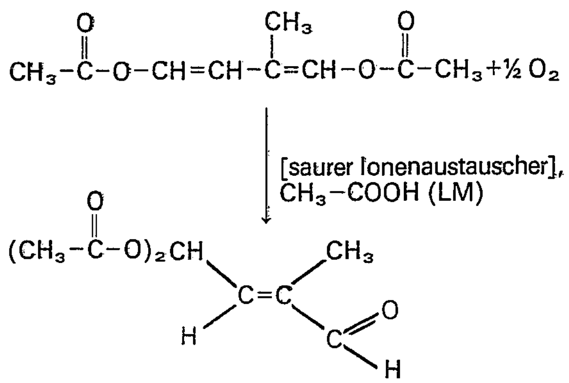


Die als Ausgangsstoffe verwendeten 2-Alkyl-1,4-diacyloxy-1,3-butadiene der Formel II enthalten als Rest $R^1$ einen Alkylrest mit 1 bis 5, vorzugsweise 1 bis 3 C-Atomen. Die beiden Reste $R^2$, die gleich oder verschieden sein können, bedeuten Wasserstoffatome, aliphatische Reste mit 1 bis 15 C-Atomen, cycloaliphatische Reste mit 5 bis 7 C-Atomen oder aromatische Reste. Aliphatische Reste sind z.B. Alkylreste, wie Methyl-, Ethyl-, n-Propyl-, i-Propyl-, n-Butyl-, i-Butyl-, tert.-Butyl-, n-Pentyl-, Palmityl- oder Stearylreste. Als cycloaliphatische Reste kommen z.B. Cyclopentyl-, Cyclohexyl- oder Cycloheptylreste in Betracht. Aromatische Reste können z.B. Phenylreste sein, die gegebenenfalls durch Alkyl- oder Halogenreste substituiert sein können.

Beispielsweise seien die folgenden Verbindungen der Formel II genannt: 2-Methyl-, 2-Ethyl-, 2-n-Propyl-, 2-Butyl-, 2-Pentyl-1,4-diacetoxy-1,3-butadien, 2-Methyl-1-propionyloxy-4-acetoxy-1,3-butadien, 2-Methyl-1-acetocy-4-palmityloxy-1,3-butadien, 2-Methyl-1-cyclohexyloxy-4-acetoxy-1,3-butadien, 2-Methyl-1-benzoyloxy-4-acetoxy-1,3-butadien.

Die Ausgangsverbindungen der Formel II lassensich z.B. durch Acetylierung von 2-Alkyl-4-

acyloxy-2-butenalen mit Acetanhydrid (J. org. Chem. *41*, 2625 (1976)) oder durch Pyrolyse von 2-Alkyl-3,4-diacetoxy-tricyclo-[4,2,1,$0^{2,5-}$]non-7-enen (Journal of Chemical Society, Chemical Communications 1974, Seiten 956 bis 957) herstellen.

Sauerstoff kann in Form von reinem Sauerstoff oder im Gemisch mit anderen Gasen wie Stickstoff, z.B. in Form von Luft, oder mit anderen inerten Gasen wie Kohlendioxid verwendet werden. Als Sauerstoff übertragende Verbindungen sind z.B. solche geeignet, die für die Epoxidierung von Olefinen benutzt werden, wie Wasserstoffperoxid, Anthrachinonperoxide, Persäuren, wie Perameisensäure, Peressigsäure, Perpropionsäure, Perbenzoesäure, m-Chlorperbenzoesäure, Per-n-buttersäure, Per-iso-buttersäure und organische Hydroperoxide wie tert.-Butylhydroperoxid oder Cumolhydroperoxid. Verbindungen dieser Art sind z.B. in Ullmanns Encyclopädie der Technischen Chemie, 4. Auflage, Band 10, Seiten 563 bis 567 genannt. Der Sauerstoff und die Sauerstoff übertragende Verbindung können auch in Gegenwart von Epoxidationskatalysatoren verwendet werden.

Als saure Ionenaustauscher verwendet man Kationenaustauscher in ihrer Säureform. Das sind z.B. Austauscher, die aus Styrol und Divinylbenzol aufgebaut sind und Sulfonsäuregruppen enthalten, oder anorganische Kationenaustauscher, wie Zeolithe, Phenol- oder Polystyrolsulfonsäureharze, Styrolphosphonsäureharze, Styrolphosphinsäureharze, oder entsprechende saure Harze enthaltende Austauscher, z.B. bifunktionelle Kondensationsharze. Kationenaustauscher der genannten Art sind z.B. die im Handel unter den Bezeichnungen ®Lewatit S 100, ®Amberlit IR-120, ®Lewasorb, ®Dowex 50 WX 8, ®Amberlyst 15 erhältlichen Produkte. Besonders bevorzugt sind hierbei stark saure Ionenaustauscher.

Die Menge der Kationenaustauscher hängt von der Selektivität bzw. der Zahl der austauschaktiven Gruppen der verwendeten Austauscher bei der Reaktionstemperatur ab. Im allgemeinen verwendet man 0,5 bis 40 Gew.%, vorzugsweise 3 bis 25 Gew.%, Austauscher, bezogen auf die Ausgangsstoffe II.

Es ist grundsätzlich auch möglich, die bei der Umsetzung der 2-Alkyl-1,4-diacyloxy-1,3-butadiene mit Sauerstoff oder Sauerstoff übertragenden Verbindungen erhaltenen Reaktionsgemische nachträglich zusammen mit Ionenaustauschern zu erhitzen. Die so erzielbaren Ausbeuten an 2-Alkyl-4,4-diacyloxy-2-butenalen und der Gehalt an trans-2-Alkyl-4,4-diacyloxy-2-butenalen sind jedoch etwas niedriger als bei der gleichzeitigen Umsetzung.

Als Lösungsmittel kommen z.B. Carbonsäuren, wie Ameisensäure, Essigsäure, Propionsäure, Buttersäure, Valeriansäuren, Caprinsäuren, Laurinsäure, Ölsäure, Palmitinsäure, Cyclohexancarbonsäure, Benzoesäure oder Phenylessigsäure in Betracht, Essigsäure ist aus wirtschaftlichen Gründen besonders bevorzugt. Die Carbonsäure wird im allgemeinen im Überschuss, z.B. in der 1- bis 80molaren Menge, bezogen auf das eingesetzte 1,3-Dien II angewandt. Im allgemeinen verwendet man die den Acyloxygruppen zugrunde liegende Carbonsäure. Anstelle von Carbonsäuren lassen sich auch andere, unter den Reaktionsbedingungen inerte Lösungsmittel verwenden. Als Lösungsmittel dieser Art kommen z.B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole, Ether, wie Diethylether, Tetrahydrofuran oder Dioxan in Betracht. Pro Mol Ausgangsverbindung II verwendet man zweckmässigerweise 0,1 bis 80 Mol, insbesondere 2 bis 60 Mol des unter den Reaktionsbedingungen inerten Lösungsmittels.

Das neue Verfahren wird z.B. so durchgeführt, dass man pro Mol der Ausgangsverbindung II 0,5 bis 10 Mol, insbesondere 1 bis 3 Mol Sauerstoff oder der Sauerstoff übertragenden Verbindung anwendet. Man arbeitet zweckmässig bei Temperaturen zwischen 0 und 200° C, insbesondere bei 20 bis 120° C. Man wendet z.B. Sauerstoffdrucke von 1 bis 100, insbesondere 1 bis 20 bar an.

Das Verfahren kann chargenweise oder kontinuierlich, drucklos oder unter Druck durchgeführt werden. Unumgesetzte 1,3-Diene II können gegebenenfalls nach der Umsetzung destillativ von den entstandenen 2-Alkyl-4,4-diacyloxy-2-butenalen abgetrennt und erneut für die erfindungsgemässe Umsetzung verwendet werden.

Man kann die erfindungsgemässe Umsetzung z.B. so durchführen, dass man den Ionenaustauscher in einer Lösung der Ausgangsverbindung II in dem jeweiligen Lösungsmittel suspendiert und bei der jeweiligen Reaktionstemperatur mit Sauerstoff oder einer Sauerstoff übertragenden Verbindung umsetzt. Nach Beendigung der Umsetzung wird der Ionenaustauscher abfiltriert oder abzentrifugiert; das Reaktionsgemisch wird z.B. destillativ aufgearbeitet. Es ist aber auch möglich, eine Lösung der Ausgangsverbindung II über einen fest angeordneten Ionenaustauscher zu leiten und das Reaktionsgemisch anschliessend aufzuarbeiten.

Die nach dem erfindungsgemässen Verfahren mit hoher Selektivität zugänglichen trans-2-Alkyl-4,4-diacyloxy-2-butenale stellen wertvolle Zwischenprodukte für die Herstellung von Terpenen, wie Retinal, β-Carotin (DE-OS 23 57 810) und Apocarotinoiden dar.

*Beispiel 1:*

In einer Lösung aus 184 g 2-Methyl-1,4-diacetoxy-1,3-butadien, 357 g Acetanhydrid und 1000 g Eisessig wurden 10 g des unter der Bezeichnung ®Amberlite IR-120 (H-Form) im Handel erhältlichen sauren Ionenaustauschers suspendiert. Der Ionenaustauscher wurde vor seiner Verwendung mit Eisessig wasserfrei gewaschen. Das Reaktionsgemisch wurde bei 100±2° C unter Rühren innerhalb von 55 Minuten mit 81,6 g Wasserstoffperoxid (50%) versetzt und anschliessend weitere 35 Minuten bei dieser Temperatur gerührt. Perverbindungen waren nach dieser Zeit durch iodometrische Titration nicht mehr nachweisbar. Nach dem Abkühlen des Ansatzes wurde der Ionenaustauscher abfiltriert. Essigsäure und Acetanhydrid wurden am Rotationsverdampfer (70° C/ 25 mbar), Leichtsieder an einem Sambay (90° C/ 5 mbar) abdestilliert. Das Reaktionsgemisch wurde einer Sambay-Destillation (150° C/0,7 mbar) unterworfen. Hierbei wurden 110 g 2-Methyl-diacetoxybutenale (55%, bezogen auf eingesetztes 2-Methyl-1,4-diacetoxy-1,3-butadien) erhalten.

Aus dem $^1$H-NMR-Spektrum ($CDCl_3$ als Lösungsmittel) war anhand der Signale für die Aldehydprotonen der verschiedenen 2-Methyldiacetoxybutenale erkennbar, dass das Verhältnis von trans- zu cis-2-Methyl-4,4-diacetoxy-2-butenal 92:8 betrug. Ausgangsprodukt und 2-Methyl-2,4-diacetoxy-3-butenale waren nicht enthalten.

*Vergleichsbeispiel 1*

Es wurde wie in Beispiel 1 gearbeitet, wobei die Umsetzung jedoch in Abwesenheit des Ionenaustauschers durchgeführt wurde. Die Ausbeute an Methyldiacetoxy-butenalen betrug 59% (bezogen auf eingesetztes 2-Methyl-1,4-diacetoxy-1,3-butadien). Das Verhältnis von trans- (Aldehydproton: 9,45 ppm) zu cis-2-Methyl-4,4-diacetoxy-2-butenal (Aldehydproton: 10,25 ppm) war 74:26. Ausserdem enthielt das 2-Methyldiacetoxybutenal-Gemisch 7% trans- (Aldehydproton: 9,28 ppm) und 10% cis-2-Methyl-2,4-diacetoxy-3-butenal (Aldehydproton: 9,37 ppm) (bezogen auf das 2-Methyl-diacetoxybutenale). Ausgangsprodukt war nicht enthalten.

*Beispiel 2:*

Einem Autoklaven aus ®Hastelloy mit Flügelrührer (1.000 Umdrehungen/Minute) und einem freien Volumen von 0,3 l, der in einem von der Reaktionslösung durchströmten Behälter mit siebartigen Wänden 100 g ®Amberlite IR 120 (H-Form) enthielt, wurden bei einer Innentemperatur von 85±2° C und einem Gesamtdruck von 2 bar pro Stunde 200 cm³ einer Eisessiglösung mit einem Gehalt von 10 Gew.% 2-Methyl-1,4-diacetoxy-1,3-butadien und 32 l Luft zugeführt. Nach 132 Stunden Reaktionszeit unter den angegebenen Bedingungen wurde der in den folgenden 12 Stunden anfallende Reaktionsaustrag (2.440 g) aufgefangen und aufgearbeitet. Hierzu wurden Essigsäure und Leichtsieder bei 60° C/ 20 mbar am Rotationsverdampfer und 90° C/ 7 mbar am Sambay-Verdampfer abgetrennt. Der verbleibende Rückstand wurde bei 150° C/1 mbar nacheinander zweimal einer Sambay-Destillation unterworfen. Hierbei wurden 130 g 2-Methyldiacetoxybutenale (50%, bezogen auf eingesetztes 2-Methyl-1,4-diacetoxy-1,3-butadien) erhalten.

Aus dem $^1$H-NMR-Spektrum ($CDCl_3$ als Lösungsmittel) war anhand der Signale für die Aldehydprotonen der verschiedenen 2-Methyldiacetoxybutenale erkennbar, dass das Verhältnis von trans- zu cis-2-Methyl-4,4-diacetoxy-2-butenal 88:12 betrug. Ausgangsprodukt und 2-Methyl-2,4-diacetoxy-3-butenale waren nicht enthalten.

*Vergleichsbeispiel 2*

Es wurde wie im Beispiel 2 gearbeitet, wobei die Umsetzung jedoch in Abwesenheit des Ionenaustauschers durchgeführt wurde. Die Ausbeute an Methyldiacetoxy-butenalen betrug 50% (bezogen auf eingesetztes 2-Methyl-1,4-diacetoxy-1,3-butadien). Das Verhältnis von trans- (Aldehydproton: 9,45 ppm) zu cis-2-Methyl-4,4-diacetoxy-2-butenal (Aldehydproton: 10,25 ppm) war 82:18. Ausserdem enthielt das 2-Methyldiacetoxybutenal-Gemisch 18% trans-(Aldehydproton: 9,28 ppm) und 17% cis-2-Methyl-2,4-diacetoxy-3-butenal (Aldehydproton: 9,37 ppm) (bezogen auf 2-Methyl-diacetoxybutenale). Ausgangsprodukt war nicht enthalten.

**Patentansprüche**

1. Verfahren zur Herstellung von 2-Alkyl-4,4-diacyloxy-2-butenalen der Formel

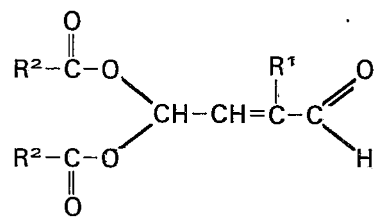

I,

in der $R^1$ einen Alkylrest mit 1 bis 5 Kohlenstoffatomen und $R^2$ ein Wasserstoffatom, einen aliphatischen Rest mit 1 bis 15 Kohlenstoffatomen, einen cycloaliphatischen Rest mit 5 bis 7 Kohlenstoffatomen oder einen aromatischen Rest bedeuten, durch Umsetzung von 2-Alkyl-1,4-diacyloxy-1,3-butadienen der Formel

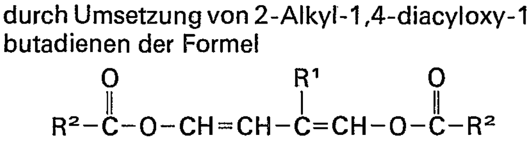

II,

in der $R^1$ und $R^2$ die obengenannte Bedeutung besitzen, in einem Lösungsmittel mit Sauerstoff oder Sauerstoff übertragenden Verbindungen, *dadurch gekennzeichnet*, dass man die Umsetzung in gegenwart von sauren Ionenaustauschern durchführt.

2. Verfahren nach Anspruch 1, *dadurch gekennzeichnet*, dass man pro Mol Ausgangsverbindung der Formel II 0,5 bis 10 Mol Sauerstoff oder Sauerstoff übertragende Verbindung anwendet.

3. Verfahren nach Anspruch 1, *dadurch gekennzeichnet*, dass man als Sauerstoff übertragende Verbindung Wasserstoffperoxid, Anthrachinonperoxide, Persäuren oder organische Hydroperoxide verwendet.

4. Verfahren nach Anspruch 1, *dadurch gekennzeichnet*, dass man als saure Ionenaustauscher Kationenaustauscher in ihrer Säureform verwendet.

5. Verfahren nach Anspruch 1, *dadurch gekennzeichnet*, dass man den Ionenaustauscher in einer Menge von 0,5 bis 40 Gew.%, bezogen auf den Ausgangsstoff II, anwendet.

## Claims

1. A process for the preparation of a 2-alkyl-4,4-diacyloxy-but-2-enal of the formula

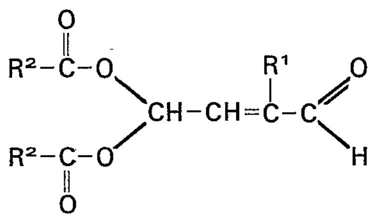

I,

where $R^1$ is alkyl of 1 to 5 carbon atoms and $R^2$ is hydrogen, an aliphatic radical of 1 to 15 carbon atoms, a cycloaliphatic radical of 5 to 7 carbon atoms or an aromatic radical, by reacting a 2-alkyl-1,4-diacyloxy-1,3-butadiene of the formula

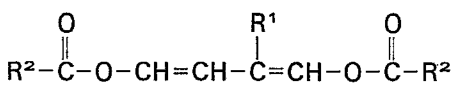

II,

where $R^1$ and $R^2$ have the above meanings, in a solvent, with oxygen or an oxygen donor, wherein the reaction is carried out in the presence of an acidic ion exchanger.

2. A process as claimed in claim 1, wherein from 0,5 to 10 moles of oxygen or an oxygen donor are used per mole of starting compound of the formula II.

3. A process as claimed in claim 1, wherein the oxygen donor used is hydrogen peroxide, an anthraquinone peroxide, a per acid or an organic hydroperoxide.

4. A process as claimed in claim 1, wherein the acidic ion exchanger used is a cation exchanger in its acid form.

5. A process as claimed in claim 1, wherein the ion exchanger is used in an amount of from 0.5 to 40% by weight, based on the starting material II.

## Revendications

1. Procédé de préparation de 2-alkyl-4,4-diacyloxy-2-buténals de la formule

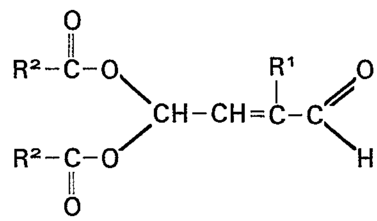

(I),

dans laquelle $R^1$ désigne un radical alkyle en $C_1$ à $C_5$ et $R^2$ un atome d'hydrogène, un groupe aliphatique en $C_1$ à $C_{15}$, un groupe cycloaliphatique en $C_5$ à $C_7$ ou un groupe aromatique, par réaction de 2-alkyl-1,4-diacyloxy-1,3-butadiènes de la formule

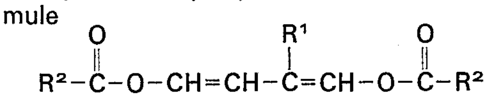

(II),

dans laquelle $R^1$ et $R^2$ possèdent les significations définies, dans un solvant avec l'oxygène ou des composés libérant de l'oxygène, caractérisé en ce que la réaction est réalisée en présence d'échangeurs d'ions acides.

2. Procédé suivant la revendication 1, caractérisé en ce que l'on met en œuvre 0,5 à 10 moles d'oxygène ou d'un composé libérant de l'oxygène par mole de composé de départ de la formule II.

3. Procédé suivant la revendication 1, caractérisé en ce que les composés libérant de l'oxygène sont choisis parmi le peroxyde d'hydrogène, les peroxydes d'anthraquinone, les per-acides et les hydroperoxydes organiques.

4. Procédé suivant la revendication 1, caractérisé en ce que l'échangeur d'ions acide est un échangeur de cations dans sa forme acide.

5. Procédé suivant la revendication 1, caractérisé en ce que l'échangeur d'ions est mis en œuvre à raison de 0,5 à 40% en poids par rapport au composé de départ de la formule II.